(19) Europäisches Patentamt
European Patent Office
Office européen des brevets

(11) **EP 3 949 839 A1**

(12) **EUROPEAN PATENT APPLICATION**
published in accordance with Art. 153(4) EPC

(43) Date of publication:
**09.02.2022 Bulletin 2022/06**

(21) Application number: **20777510.7**

(22) Date of filing: **24.03.2020**

(51) International Patent Classification (IPC):
**A61B 5/021** $^{(2006.01)}$

(52) Cooperative Patent Classification (CPC):
**A61B 5/021**

(86) International application number:
**PCT/JP2020/012926**

(87) International publication number:
**WO 2020/196488 (01.10.2020 Gazette 2020/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.03.2019 JP 2019058770**

(71) Applicant: **Medical Photonics Co., Ltd.
Sapporo-shi, Hokkaido 001-0021 (JP)**

(72) Inventor: **IINAGA, Kazuya
Sapporo-shi, Hokkaido 001-0021 (JP)**

(74) Representative: **Vossius & Partner
Patentanwälte Rechtsanwälte mbB
Siebertstraße 3
81675 München (DE)**

(54) **BLOOD PRESSURE MEASUREMENT DEVICE AND METHOD THEREFOR**

(57) [Problem] To provide a device for measuring blood pressure by non-invasive lipid measurement.

[Solution] The present invention has: an irradiation unit for irradiating periodic light onto a subject having a first layer on the surface and a second layer underneath the first layer that absorbs more light than the first layer; a light intensity detection unit disposed at a distance from the irradiation unit at which the periodic light passes through the first layer and the second layer and is detected as light that has lost periodicity, the light intensity detection unit detecting the intensity of the light that has lost periodicity emitted from the subject; and a control unit for calculating blood pressure in the subject from the intensity of the light that has lost periodicity.

FIG. 1

EP 3 949 839 A1

**Description**

Technical Field

The present invention relates to a device for measuring blood pressure and a method therefor.

Background Art

In light-based biometrics of related art, a variety of analytic innovations have been made to take advantage of two phenomena of absorption and scattering.

For example, Patent Literature 1 shows a method for deriving the scattering and absorption coefficients in a single measurement operation.

Citation List

Patent Literature

Patent Literature 1: International Publication No. 2014/087825

Summary of Invention

Technical Problem

The approach shown in Patent Literature 1, however, assumes that any subject is a uniform layer, so that further improvement in accuracy is required in measurement of a biological body formed of a plurality of layers. Furthermore, improvement in the accuracy of the biometrics may lead to possibility of blood pressure measurement.

The present invention has been made to solve the problem with the related art and provides a device and a method for measuring blood pressure based on noninvasive lipid measurement.

Solution to Problem

A blood pressure measurement device according to the present invention includes a light radiator that radiates light having periodicity to a subject having a first layer at a surface of the subject and a second layer that is located underneath the first layer and absorbs light by a greater amount than the first layer, a light intensity detector that is disposed at a distance over which the light having periodicity passes from the light radiator through the first and second layers and is detected as light having lost the periodicity, the light intensity detector detecting intensity of the light emitted from the subject and having lost the periodicity, and a controller that calculates blood pressure in the subject from the intensity of the light having lost the periodicity.

A blood pressure measurement device according to the present invention is a blood pressure measurement device communicably connected to a user device including a light radiator that radiates light having periodicity to a subject having a first layer at a surface of the subject and a second layer that is located underneath the first layer and absorbs light by a greater amount than the first layer and a light intensity detector that is disposed at a distance over which the light having periodicity passes from the light radiator through the first and second layers and is detected as light having lost the periodicity, the light intensity detector detecting intensity of the light emitted from the subject and having lost the periodicity, and the blood pressure measurement device includes a controller that calculates blood pressure in the subject from the intensity of the light having lost the periodicity and transmitted from the user device.

A blood pressure measurement method according to the present invention includes radiating light having periodicity to a subject having a first layer at a surface of the subject and a second layer that is located underneath the first layer and absorbs light by a greater amount than the first layer, detecting intensity of the light emitted from the subject and having lost the periodicity in a position where the light having periodicity passes from the radiation position through the first and second layers and is detected as light having lost the periodicity, and calculating blood pressure in the subject from the intensity of the light having lost the periodicity.

Advantageous Effects of Invention

The blood pressure measurement device and a method therefor according to the present invention allow blood pressure measurement.

Brief Description of Drawings

[Figure 1] Figure 1 shows the periodicity of light.
[Figure 2] Figure 2 shows disturbed periodicity of light.
[Figure 3] Figure 3 shows a two-layer system.
[Figure 4] Figure 4 shows the correlation between a wave height and blood pressure.
[Figure 5] Figure 5 shows an example of the configuration of a blood pressure measurement device according to an embodiment.
[Figure 6A] Figure 6A shows an example in which a light intensity detector is disposed on the opposite surface.
[Figure 6B] Figure 6B shows another example in which the light intensity detector is disposed on the opposite surface.
[Figure 7] Figure 7 is a block diagram of the blood pressure measurement device according to the embodiment.
[Figure 8] Figure 8 shows an example of the configuration of the blood pressure measurement device according to another embodiment.
[Figure 9] Figure 9 is a flowchart of a blood pressure measurement method according to the embodiment.

Description of Embodiment

The measurement principle employed by a blood pressure measurement device and a method therefor according to an embodiment will first be described.

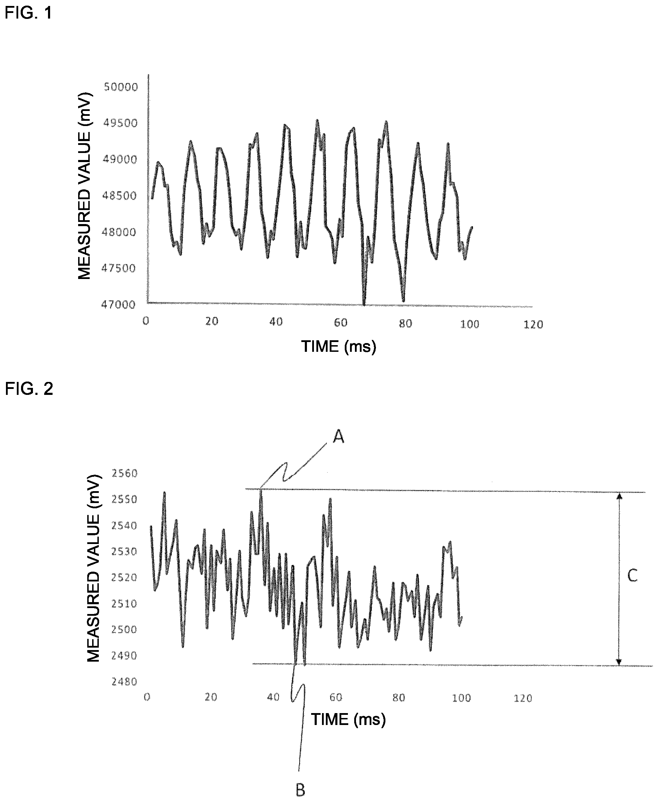

Periodic light, such as light from a fluorescent lamp, emits as light having periodicity, and the intensity of the received light also changes accordingly, as shown in Figure 1.

When periodic light enters a scattering absorber, the light loses its periodicity, as shown in Figure 2. The difference (C in Figure 2) between the maximum (A in Figure 2) and the minimum (B Figure 2) of the intensity of the received incident light within a predetermined period is called a wave height. The periodicity appears to be cluttered at first glance. Although the periodicity appears to be lost, the photons least affected by absorption of the absorbing scatterer provide the maximum and minimum, and the photons lost due to the absorption appear in the form of the cluttered state.

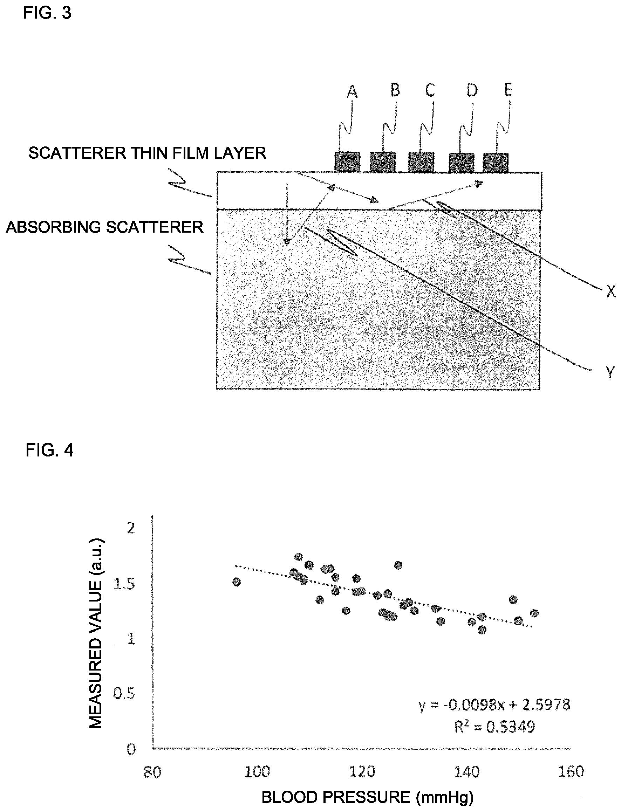

The phenomenon described above is applicable, for example, to the two-layer system shown in Figure 3, which is formed of a scatterer thin film layer (first layer) and an absorbing scatterer (second layer) underneath the scatterer thin film layer. In the case of two layers having different refractive indices, incident light X diffuses in the first layer, so that the amount of light that enters the second layer decreases.

That is, a light receiving section A receives two types of light: light that has only been scattered but has not been absorbed; and light that has been both scattered and absorbed.

In actual measurement, the first and second layers can be regarded as layers containing blood and containing no blood, such as a capillary layer. For example, the first layer is the epidermis, and the second layer is the dermis.

When the distance between the light incident section and the light receiving section is relatively short, as in the case of the light receiving section A, measured values are affected by the light absorption of the absorbing scatterer due to the cluttered waveform and the decrease in the wave height and measurement values. In the wave height analysis at such a short distance, the light is reflected at an acute angle backward, and absorption is involved. It is therefore believed that the blood cells, which are absorption scatterers larger than the wavelength of the light, greatly affect measured values.

In view of the fact described above, the present inventor has examined the correlation between the wave height (difference C in measured intensity of received light in Figure 2) and the blood pressure in the case where the distance between the light incident section and the light receiving section is relatively short, such as the case of the light receiving section A in Figure 3. The reason for this is that the blood pressure is assumed to be maximum pressure and the blood ratio in the optical path is believed to have been increased due, for example, to indirect capillary dilation.

The short distance between the light incident section and the light receiving section used herein refers to an area where the incident light having lost its periodicity is detected. For example, the distance between the light incident section and the light receiving section is a distance at which the intensity of the received light is at least 1/20 of the intensity of the incident light or a distance at which the light has lost its periodicity and therefore the periodicity cannot be ascertained by FFT analysis. For example, the periodicity of light can be ascertained by FFT analysis as follows: When the measured intensity is at least 1.1 times greater than the intensity at a target frequency in the FFT analysis plus or minus 10 Hz (that is, measurable FFT intensity ratio > (intensity at target frequency in FFT) / {[(intensity at target frequency +10 Hz in FFT) + (intensity at target frequency - 10 Hz in FFT)] / 2} = 1.1)), the periodicity is maintained. Conversely, the periodicity of light can be ascertained by FFT analysis as follows: When the measured intensity is 1.1 times smaller than or equal to the intensity at a target frequency in the FFT analysis plus or minus 10 Hz, the periodicity has been lost.

Figure 4 shows the correlation between the wave height and the blood pressure. Optical measurement and blood pressure measurement were performed on six subjects six times a day, as shown in Figure 4. As a result, the value of the correlation is greater than or equal to 0.7, which ascertains that the blood pressure was successfully measured.

The wave height is measurable even by only-one-point measurement, but measured values are likely to contain errors because the measured values involve the absorption and the scattering. It is therefore desirable to smooth out the errors in the measurement based, for example, on the rate of change in wave height intensity detected when the light is received at a plurality of points at the same distance or when the distance is changed.

The wave height only needs to be found in terms of amplitude and may instead be the median or the average of measured wave heights or the width between the maximum and the minimum in the measurement period. The wave height may instead be analyzed by using the difference between the average of the measured wave heights and the peak top thereof, or the difference between the average of the measured wave heights and the peak bottom thereof, that is, the half of the measured wave heights.

An embodiment will be described below with reference to the drawings.

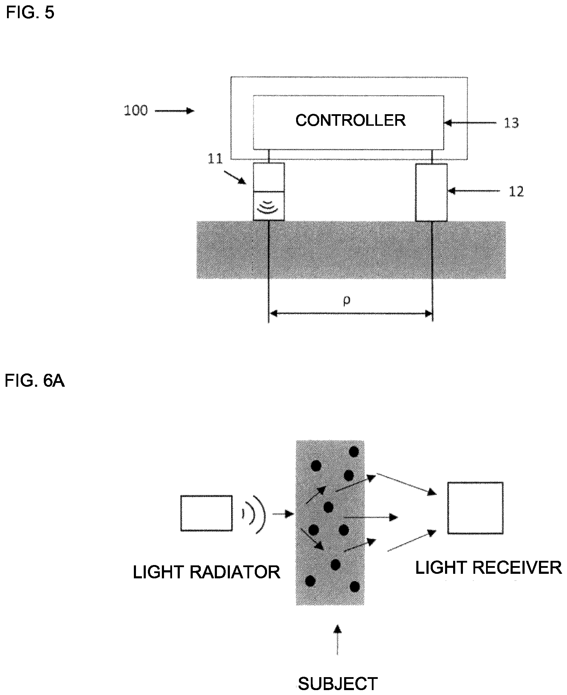

Figure 5 schematically shows an example of the configuration of a blood pressure measurement device 100 according to the embodiment. The blood pressure measurement device 100 includes a light radiator 11, a light intensity detector 12, and a controller 13, as shown in Figure 5.

The light radiator 11 in the embodiment is disposed at a predetermined distance $\rho$ from the light intensity detector 12. The light radiator 11 radiates light having periodicity to a subject C, which has a first layer at the surface of the subject C and a second layer that is located underneath the first layer and absorbs light by a greater amount than the first layer. The light radiator 11 is, for example, a fluorescent lamp, an LED, a laser, an incandescent lamp, an HID, or a halogen lamp. The light radiator 11 may be provided with a mechanism that periodically opens and closes, such as a shutter, to adjust a light blocking period so as to provide the continuously radiated light with periodicity. The illuminance of the light from the light radiator 11 is controlled by the controller 13.

The light radiator 11 can adjust the range of the wavelength in such a way that the wavelength range does not fall within the range of the wavelengths at which the light is absorbed by inorganic substances of the blood plasma. The light radiator 11 can perform the adjustment in such a way that the wavelength range does not fall within the range of the wavelengths at which the light is absorbed by the cell components of the blood. The cell components of the blood used herein are the red blood cells, white blood cells, and platelets in the blood.
The inorganic substances of the blood plasma are water and electrolytes in the blood.

It is assumed in the embodiment that the blood pressure measurement device 100 includes the light radiator 11, which is a light source that outputs light having periodicity, and light from a fluorescent lamp, an LED illuminator, or any other light source in the room where the blood pressure measurement device 100 is installed may be used. In this case, it is not necessary to provide the blood pressure measurement device 100 with the light radiator 11.

The light intensity detector 12 in the embodiment receives light emitted from the interior of the subject C to the exterior of the subject C. The light intensity detector 12 is disposed at the distance $\rho$ from the light radiator 11 over which the light having periodicity passes through the first and second layers of the subject C and is detected as light having lost its periodicity. The light intensity detector 12 in the embodiment is a photodiode. The light intensity detector 12 is not limited to a photodiode and may instead be a CCD or a CMOS device. The light intensity detector 12 detects the intensity of the light emitted from the subject and having lost its periodicity. The light intensity detector 12 may be a component capable of receiving light having a wavelength so set as to belong to light other than the visible light. The light intensity detector 12 is controlled by the controller 13. The light intensity detector 12 transmits the sensed light intensity to the controller 13.

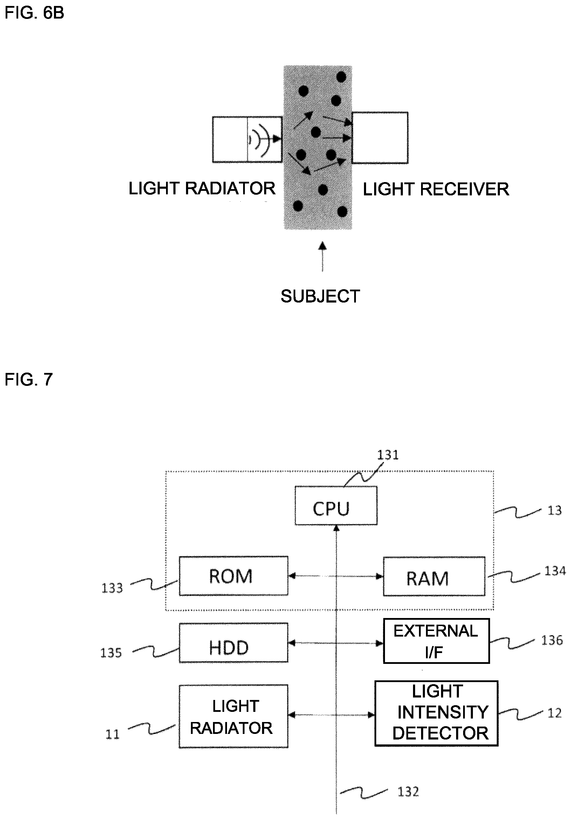

The light intensity detector 12 (light receiver in the figures) may be disposed on the opposite side of the subject from the light radiator 11, as shown in Figures 6A and 6B. This arrangement can be employed when the measurement is performed at a location which is not relatively very thick and through which light readily passes, such as an earlobe and a fingertip. The light radiator and the light intensity detector (light receiver in the figures) may (Figure 6B) or may not (Figure 6A) be in contact with a subject under detection.

The configuration of a control system of the blood pressure measurement device 100 will next be described. Figure 7 is a block diagram of the blood pressure measurement device 100 according to the embodiment. A CPU (central processing unit) 131, a ROM (read only memory) 133, a RAM (random access memory) 134, an HDD (hard disk drive) 135, an external I/F (interface) 136, the light radiator 11, and the light intensity detector 12 are connected to each other via a system bus 132. The CPU 131, the ROM 133, and the RAM 134 form the controller 13.

The ROM 133 stores in advance programs executed by the CPU 131 and thresholds used by the CPU 131.

The RAM 134 has an area where the programs executed by the CPU 131 are developed, a variety of memory areas, such as a work area where the programs process data, and other areas.

The HDD 135 stores data on a calibration curve created by correlating the blood pressure to the wave height for a plurality of persons.

The external I/F 136 is an interface for communication with an external device, for example, a client terminal (PC). The external I/F 136 only needs to be an interface for data communication with the external device, for example, may be an apparatus (such as USB memory) to be locally connected to the external device or a network interface for communication via a network.

The blood pressure measurement device 100 having the configuration described above performs a blood pressure measurement job based on a program set in advance.

The controller 13 calculates the wave height from the intensity of the light having lost its periodicity and sensed by the light intensity detector 12.

The wave height can be calculated by the following expression:

$$\text{Wave height = received light intensity tp (mV)} -$$
$$\text{received light intensity tb (mV)}$$

The received light intensity tp (mV) represents the received light intensity at the peak top of a received light intensity change that occurs when the illumination light periodically changes in a predetermined period. In the received light intensity change for 100 ms, A in Figure 2 corresponds to the peak-top received light intensity. The received light intensity tb (mV) represents the received light intensity at the bottom of the periodic change in the received light intensity in the predetermined period. In the received light intensity change for 100 ms, B in Figure 2 corresponds to the bottom received light intensity. The character C in Figure 2 therefore represents the wave height in the received light intensity change for 100 ms. The character tp represents the point of time when the received light intensity change reaches the peak top. The symbol tb represents the point of time when the received light intensity change reaches the bottom. In the embodiment, the predetermined period is 100 ms, but not necessarily, and may be about 100 ms or any other period.

For example, when room light is used and a plurality of light sources are present, a waveform with a shoulder is formed, for example, due to the differences in intensity of the light radiated from the light sources. Also in this case, the peak top and the peak bottom in the measurement may be employed, as described above.

To calculate the amount of periodic change, the difference between the average and the peak top or the difference between the average and the peak bottom, that is, a half the maximum intensity may be used to perform the analysis of the wave height.

The controller 13 calculates the blood pressure based on the wave height (difference between maximum and minimum of intensity of received light having lost its periodicity in predetermined period). The calculation method includes creating correlation between the wave height and the blood pressure for a plurality of persons, such as the correlation shown in Figure 4, holding data on the correlation in the form of a calibration curve in the HDD 135, and causing the controller 13 to calculate the blood pressure corresponding to the wave height from the data. The difference between the average and the peak top or between the average and the peak bottom of the received light intensity in the predetermined period, that is, a half the maximum intensity may be used to correlate the wave height with the blood pressure.

In the embodiment, the light radiator, the received light intensity detector, and the controller are integrated with one another into a single device, but not necessarily. For example, an illuminator (such as LED) and a sensor (such as CMOS device) provided in a user device, such as a mobile terminal (smartphone, tablet terminal, and PC) as the light radiator and the light intensity detector may be used, and the controller may be installed in a server device connected to the user device over a network.

A blood pressure measurement device according to the embodiment is communicably connected to a user device including a light radiator that radiates light having periodicity to a subject having a first layer at the surface of the subject and a second layer that is located underneath the first layer and absorbs light by a greater amount than the first layer and a light intensity detector that is disposed at a distance over which the light radiated from the light radiator and having periodicity passes through the first and second layers and is detected as light having lost its periodicity, the light intensity detector detecting the intensity of the light emitted from the subject and having lost its periodicity. The blood pressure measurement device includes a controller that determines the wave height from the intensity of the light having lost its periodicity and transmitted from the user device and calculates the blood pressure in the subject from the wave height. The contents of specific processes carried out by the blood pressure measurement device are the same as the contents of the processes carried out by the blood pressure measurement device according to the embodiment described above and are therefore not described.

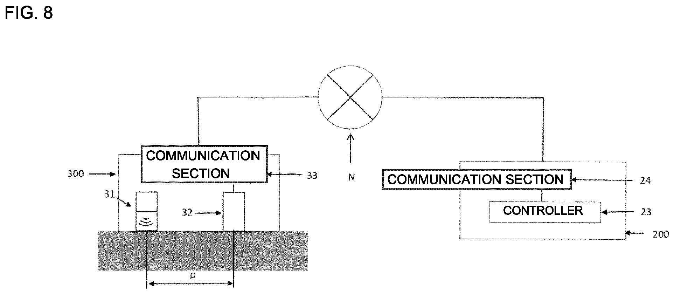

Figure 8 shows the configuration of the blood pressure measurement device according to another embodiment.

The blood pressure measurement system according to the embodiment is formed of a user device 300, which measures the light intensity, and a blood pressure measurement device 200, which calculates the blood pressure based on the light intensity. The user device 300 and the blood pressure measurement device 200 are connected by a network to each other over a wireless or wired communication network N.

The blood pressure measurement device 200 is a device for calculating the blood pressure by carrying out a predetermined process based on the light intensity transmitted from the user device 300. Specifically, as the blood pressure measurement device 200, a personal computer is used or a server device is used depending on the number of devices and the amount of data to be transmitted and received as appropriate.

The user device 300 is a device carried by a user and is a standalone device in some cases or is incorporated in a mobile phone, a wristwatch, or any other apparatus in other cases.

The user device 300 includes a light radiator 31, which radiates light having periodicity to a subject having a first layer at the surface of the subject and a second layer that is located underneath the first layer and absorbs light by a greater amount than the first layer, a light intensity detector 32, which detects the intensity of the light having periodicity and having lost its periodicity after passing through the first and second layers, and a communication section 33. The communication section 33 transmits the intensity of the light having lost its periodicity and detected by the light intensity detector 32. The actions and functions of the light radiator 31 and the light intensity detector 32 are the same as those in the embodiment described above.

The blood pressure measurement device 200 includes a communication section 24 and a controller 23. The communication section 24 receives the intensity of the light having lost its periodicity and transmitted from the communication section 33 over the wired or wireless network N and transmits the received light intensity to the controller 23. The action and function of the controller 23 are the same as those of the controller 13 in the embodiment described above.

In the present embodiment, the intensity of the light having lost its periodicity is transmitted from the user device 300 to the blood pressure measurement device 200 over the network N, but not necessarily, and the user device 300 and the blood pressure measurement device 200 may be directly connected to each other without the network N and may transmit the light intensity, for example, over wired or wireless communication.

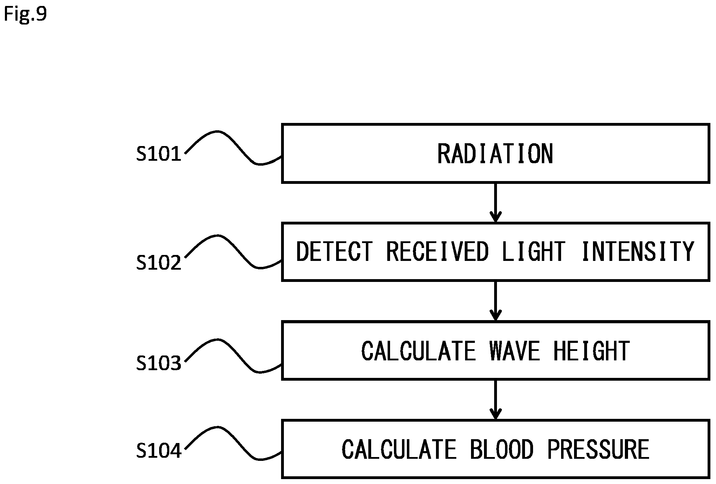

A blood pressure measurement method according to the embodiment will next be described. Figure 9 is a flowchart of the blood pressure measurement method according to the embodiment.

The blood pressure measurement method according to the embodiment includes causing a light radiator to radiate light having periodicity to a predetermined radiation position on a subject having a first layer at the surface of the subject and a second layer that is located underneath the first layer and absorbs light by a greater amount than the first layer (STEP 101), causing a light intensity detector to detect the intensity of the light emitted from the subject and having lost its periodicity in a position where the light having periodicity passes from the radiation position through the first and second layers and is detected as light having lost its periodicity (STEP 102), causing a controller to determine the wave height from the intensity of the light having lost its periodicity (STEP 103), and determining the blood pressure from the wave height (STEP 104). The contents of specific processes carried out by the blood pressure measurement device are the same as the contents of the processes carried out by the blood pressure measurement device according to the embodiment described above and are therefore not described.

A blood pressure measurement program according to the embodiment will next be described.

A device is communicably connected to a user device including a light radiator that radiates light having periodicity to a subject having a first layer at the surface of the subject and a second layer that is located underneath the first layer and absorbs light by a greater amount than the first layer and a light intensity detector that is disposed in a position where the light radiated from the light radiator and having periodicity passes through the first and second layers and is detected as light having lost its periodicity, the light intensity detector detecting the intensity of the light emitted from the subject and having lost its periodicity.

The blood pressure measurement program according to the embodiment causes a computer of the device to perform the process of determining the wave height from the intensity of the light having lost its periodicity and transmitted from the user device and determining the blood pressure from the wave height. The contents of specific processes in the program are the same as the contents of the processes in the embodiment described above and are therefore not described.

The embodiments have been described above but have been presented by way of example and are not intended to limit the scope of the present invention. The novel embodiments can be implemented in a variety of other forms, and a variety of omissions, replacements, and changes can be made to the embodiments to the extent that they do not depart from the substance of the present invention. The embodiments and variations thereof fall within the scope and substance of the present invention and also fall within the inventions set forth in the claims and the scope equivalent thereto.

Reference Sings list

100:    Blood pressure measurement device
11:    Light radiator
12:    Received light intensity detector

13: Controller

**Claims**

1. A blood pressure measurement device comprising:

    a light radiator that radiates light having periodicity to a subject having a first layer at a surface of the subject and a second layer that is located underneath the first layer and absorbs light by a greater amount than the first layer;
    a light intensity detector that is disposed at a distance over which the light having periodicity passes from the light radiator through the first and second layers and is detected as light having lost the periodicity, the light intensity detector detecting intensity of the light emitted from the subject and having lost the periodicity; and
    a controller that calculates blood pressure in the subject from the intensity of the light having lost the periodicity.

2. The blood pressure measurement device according to claim 1, wherein the distance over which the light having periodicity is detected as light having lost the periodicity is a distance at which the intensity of the received light is at least 1/20 of the intensity of the light incident from the light radiator.

3. The blood pressure measurement device according to claim 1, wherein the distance over which the light having periodicity is detected as light having lost the periodicity is a distance at which the periodicity of the light from the light radiator cannot be ascertained by FFT analysis.

4. The blood pressure measurement device according to claim 1, wherein the controller calculates the blood pressure in the subject from a difference between a maximum and a minimum of the intensity of the light having lost the periodicity in a predetermined period.

5. The blood pressure measurement device according to claim 4, wherein the predetermined period is about 100 ms.

6. A blood pressure measurement device communicably connected to a user device including a light radiator that radiates light having periodicity to a subject having a first layer at a surface of the subject and a second layer that is located underneath the first layer and absorbs light by a greater amount than the first layer and a light intensity detector that is disposed at a distance over which the light having periodicity passes from the light radiator through the first and second layers and is detected as light having lost the periodicity, the light intensity detector detecting intensity of the light emitted from the subject and having lost the periodicity, wherein the blood pressure measurement device includes a controller that calculates blood pressure in the subject from the intensity of the light having lost the periodicity and transmitted from the user device.

7. A blood pressure measurement method comprising:

    radiating light having periodicity to a subject having a first layer at a surface of the subject and a second layer that is located underneath the first layer and absorbs light by a greater amount than the first layer;
    detecting intensity of the light emitted from the subject and having lost the periodicity in a position where the light having periodicity passes from the radiation position through the first and second layers and is detected as light having lost the periodicity; and
    calculating blood pressure in the subject from the intensity of the light having lost the periodicity.

FIG. 1

FIG. 2

FIG. 3

SCATTERER THIN FILM LAYER

ABSORBING SCATTERER

FIG. 4

$y = -0.0098x + 2.5978$
$R^2 = 0.5349$

BLOOD PRESSURE (mmHg)

FIG. 5

FIG. 6A

FIG. 6B

LIGHT RADIATOR          LIGHT RECEIVER

SUBJECT

FIG. 7

FIG. 8

Fig.9

S101 — RADIATION

S102 — DETECT RECEIVED LIGHT INTENSITY

S103 — CALCULATE WAVE HEIGHT

S104 — CALCULATE BLOOD PRESSURE

<table>
<tr><td colspan="2" align="center">**INTERNATIONAL SEARCH REPORT**</td><td colspan="2">International application No.<br>PCT/JP2020/012926</td></tr>
</table>

**A. CLASSIFICATION OF SUBJECT MATTER**
Int.Cl. A61B5/021(2006.01)i
FI: A61B5/021

According to International Patent Classification (IPC) or to both national classification and IPC

**B. FIELDS SEARCHED**

Minimum documentation searched (classification system followed by classification symbols)
Int.Cl. A61B5/021

Documentation searched other than minimum documentation to the extent that such documents are included in the fields searched
Published examined utility model applications of Japan   1922-1996
Published unexamined utility model applications of Japan   1971-2020
Registered utility model specifications of Japan   1996-2020
Published registered utility model applications of Japan   1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2006-102166 A (NIPPON TELEGRAPH AND TELEPHONE CORPORATION) 20.04.2006 (2006-04-20), entire text, all drawings | 1-7 |
| A | JP 2008-79688 A (CITIZEN HOLDINGS CO., LTD.) 10.04.2008 (2008-04-10), entire text, all drawings | 1-7 |
| A | JP 2015-39542 A (SEIKO EPSON CORPORATION) 02.03.2015 (2015-03-02), entire text, all drawings | 1-7 |
| A | JP 2010-36799 A (NIPPON SOKEN INC.) 18.02.2010 (2010-02-18), entire text, all drawings | 1-7 |
| A | JP 2006-102191 A (NIPPON TELEGRAPH AND TELEPHONE CORPORATION) 20.04.2006 (2006-04-20), entire text, all drawings | 1-7 |

☒ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>02.06.2020 | Date of mailing of the international search report<br>09.06.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2020/012926

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2006-280393 A (TERUMO CORPORATION) 19.10.2006 (2006-10-19), entire text, all drawings | 1-7 |
| A | JP 2006-102261 A (NIPPON TELEGRAPH AND TELEPHONE CORPORATION) 20.04.2006 (2006-04-20), entire text, all drawings | 1-7 |

Form PCT/ISA/210 (second sheet) (January 2015)

INTERNATIONAL SEARCH REPORT
Information on patent family members

International application No.

PCT/JP2020/012926

```
JP 2006-102166 A    20.04.2006    (Family: none)

JP 2008-79688 A     10.04.2008    (Family: none)

JP 2015-39542 A     02.03.2015    US 2015/0057508 A1
                                  entire text, all drawings
                                  EP 2839778 A1
                                  CN 104414625 A

JP 2010-36799 A     18.02.2010    US 2010/0036592 A1
                                  entire text, all drawings

JP 2006-102191 A    20.04.2006    (Family: none)

JP 2006-280393 A    19.10.2006    (Family: none)

JP 2006-102261 A    20.04.2006    (Family: none)
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087825 A